# EUROPÄISCHE PATENTSCHRIFT

(11) **EP 1 116 763 B1**
(45) Veröffentlichungstag und Bekanntmachung des Hinweises auf die Patenterteilung: **31.03.2004**
(21) Anmeldenummer: 00128030.4
(22) Anmeldetag: 21.12.2000
(51) Int. Cl.: C09J 7/00, C09J 7/02, A61L 15/44, C09J 123/22

(54) **Verfahren zur kontinuierlichen Herstellung und Beschichtung von Selbstklebemassen auf Basis von Polyisobutylen mit mindestens einem pharmazeutischen Wirkstoff**
Process for the continuous production and coating of self-adhesive masses based on polyisobutylene and at least one pharmaceutical active compound
Procédé pour la fabrication et le revêtement en continu de masses autocollantes à base de polyisobutylène avec au moins une substance pharmaceutiquement active

(30) Priorität: 14.01.2000 DE 10001546
(43) Veröffentlichungstag der Anmeldung: 18.07.2001
(73) Patentinhaber: Beiersdorf Aktiengesellschaft, 20245 Hamburg (DE)
(72) Erfinder: Wüstling, Jens-Uwe, Dr., 22589 Hamburg (DE); Wasner, Matthias, 21029 Hamburg (DE); Kirchner, Joachim, 25436 Uetersen (DE); Uphus, Reinhard, Dr., 30419 Hannover (DE); Struckmann, Sören, 31515 Steinhude (DE)

(56) Entgegenhaltungen:
- WO-A-94/11175
- WO-A-96/22083
- WO-A-99/42276
- DE-A- 2 452 584

## Beschreibung

Die vorliegende Erfindung bezieht sich auf ein Verfahren zur kontinuierlichen Herstellung und Beschichtung von Selbstklebemassen auf Basis von Polyisobutylen mit mindestens einem pharmazeutischen Wirkstoff.

Für das anwendungstechnische Anforderungsprofil von druckempfindlichen Klebersystemen und den damit hergestellten Haftklebeartikeln (wie zum Beispiel Pflaster) sind die zwei physikalischen Phänomene Adhäsion und Kohäsion der Haftkleberschichten von grundsätzlichem Inhalt. Die Adhäsion wird im technischen Sprachgebrauch unter den Begriffen Sofortklebkraft (Tack) und Klebkraft (Peelstrength) behandelt und beschreibt definitionsgemäß die Begriffe "selbstklebend", "Haftkleber" und/oder "druckempfindliche Klebebänder", d.h. die dauerhafte Verklebung unter "leichtem Andruck" ("Pressure Sensitive Adhesives").

Diese Eigenschaft wird insbesondere bei den Haftklebern auf Basis von Kautschuk durch Einmischen von klebrigmachenden Harzen (Tackifier) und Weichmachern mit relativ niedrigen Molekulargewichten erzielt.

Die zweite definitionsgemäße Eigenschaft der Haftkleber ist ihre einfache rückstandsfreie Wiederablösbarkeit nach dem Gebrauch. Dieses Verhalten ist im wesentlichen bestimmt durch die hochmolekularen Kautschukanteile als Elastomerkomponente, die dem System als Kohäsion (innere Festigkeit) die geforderte Festigkeit bei Scherbeanspruchung verleihen, was insbesondere für den Einsatz der Produkte bei mechanischer Belastungen Bedeutung erhält.

Die Leistungsfähigkeit des Haftklebers ist also im wesentlichen bestimmt durch das ausgewogene Verhältnis von Adhäsions- und Kohäsions-Eigenschaften und durch Verträglichkeit, Homogenität und Stabilität der Abmischung von Massebestandteilen mit extrem hohen und relativ niedrigen mittleren Molekulargewichten, was bei der Masseherstellung in branchenüblichen Misch- und Knetmaschinen unter Verwendung von Lösemitteln relativ einfach zu erreichen ist.

Der Vorteil des Lösungsmittelverzichts besteht im wesentlichen in der Vereinfachung des Streichprozesses. Der Verzicht auf brennbare Lösemittel macht die Trockneranlagen mit ihrem aufwendigen Energieaufwand für das Verdampfen und Rückgewinnen der Lösemittel und den Einsatz explosionsgeschützter Anlagen überflüssig. Hotmeltbeschichtungsanlagen sind kompakt und erlauben erheblich höhere Streichgeschwindigkeiten. Es handelt sich um eine umweltfreundliche Technologie, bei der keine Lösemittelemissionen auftreten. Weiterhin verbleiben so keine unerwünschten Lösungsmittelreste in der Selbstklebemasse. Dieses begründet die Senkung des allergenen Potentials des Produkts.

Für die lösungsmittelfreie Compoundierung werden gemäß dem Stande der Technik vorwiegend Blockcopolymere mit Polystyrolblockanteilen oder Natur- bzw. Synthetikkautschuke verwendet.

Durch die hochmolekularen Anteile des Kautschuks (mit M_{w} ≥ 3*10⁵ g/mol) sind lösungsmittelfreie Selbstklebemassen mit der Schmelzhaftklebertechnologie unverarbeitbar oder aber die verwendeten Kautschuke müssen vor der Verarbeitung stark in ihrem Molekulargewicht reduziert (abgebaut) werden.

Der zielgerichtet durchgeführte technische Prozeß des Kautschukabbaus unter der kombinierten Einwirkung von Scherspannung, Temperatur, Luftsauerstoff wird in der Fachliteratur als Mastikation (engl.: mastication) bezeichnet und zumeist im Beisein chemischer Hilfsmittel durchgeführt, welche aus der Fachliteratur als Mastiziermittel oder Peptizer, seltener auch als "chemische Plastiziermittel" bekannt sind.
Der Mastikationsschritt ist in der Kautschuktechnologie nötig, um eine Erleichterung der Aufnahme der Zusatzstoffe zu erzielen.

Nach Römpp (Römpp Lexikon Chemie - Version 2.0, Stuttgart/New York: Georg Thieme Verlag 1999) stellt die Mastikation eine gummitechnologische Bezeichnung für den Abbau langkettiger Kautschuk-Moleküle zur Erhöhung der Plastizität beziehungsweise Reduzierung der (Mooney-)Viskosität von Kautschuken dar. Die Mastikation wird durchgeführt durch Behandlung insbesondere von Naturkautschuk in Knetern oder zwischen Walzen bei möglichst niedrigen Temperaturen in Gegenwart von Mastikationshilfsmitteln (Mastizierhilfsmittel). Die dabei einwirkenden hohen mechanischen Kräfte führen zu einem "Zerreißen" der Kautschuk-Moleküle unter Bildung von Makroradikalen, deren Rekombination durch Reaktion mit Luftsauerstoff verhindert wird. Mastikationshilfsmittel wie aromatische oder heterocyclische Mercaptane beziehungsweise deren Zink-Salze oder Disulfide beschleunigen durch Begünstigung der Bildung von Primärradikalen den Mastikationsprozeß. Aktivatoren wie Metall- (Eisen-, Kupfer-, Cobalt-) Salze von Tetraazaporphyrinen oder Phthalocyaninen ermöglichen eine Erniedrigung der Mastikationstemperatur. Mastikationshilfsmittel werden bei der Mastikation von Naturkautschuk in Mengen von ca. 0,1 bis 0,5 Gew.-% in Form von Masterbatches eingesetzt, die eine gleichmäßige Verteilung dieser geringen Chemikalienmenge in der Kautschukmasse erleichtern.

Die Mastikation ist streng zu unterscheiden von dem sich bei allen üblichen lösungsmittelfreien Polymertechnologien wie Compoundieren, Fördern und Beschichten in der Schmelze ergebenden Abbau, der Degradation (engl.: degradation).
Die Degradation ist eine Kollektivbezeichnung für unterschiedliche Prozesse, die das Aussehen und die Eigenschaften von Kunststoffen verändern. Degradation kann zum Beispiel durch chemische, thermische, oxidative, mechanische oder biologische Einflüsse oder auch durch Strahleneinwirkung (wie (UV-)Licht) verursacht werden. Folge sind zum Beispiel Oxidation, Kettenspaltungen, Depolymerisation, Vernetzung beziehungsweise Abspaltung von Seitengruppen der Polymeren. Die Stabilität von Polymeren gegenüber einer Degradation kann durch Additive, zum Beispiel durch Zusatz von Stabilisatoren wie Antioxidantien oder Photostabilisatoren erhöht werden.

Verschiedene Wege zur lösungsmittelfreien Herstellung und Verarbeitung von Kautschuk-Haftklebem wurden beschrieben.

In dem Patent CA 698 518 wird ein Prozeß beschrieben, wie eine Masseherstellung durch Zusatz hoher Weichmacheranteile und/oder gleichzeitig starker Mastikation des Kautschuks zu erreichen ist. Zwar lassen sich nach diesem Verfahren Haftkleber mit extrem hoher Anfaßklebkraft erzielen, durch den relativ hohen Weichmacheranteil oder auch den starken Abbau der Molekularstruktur des Elastomers auf ein Molekulargewichtsmittel von M_{w} ≤ 1 Million ist die anwendergerechte Scherfestigkeit auch mit anschließender höherer Vernetzung nur eingeschränkt zu erreichen.

Die Verwendung von Polymerblends, wo neben nicht-thermoplastischem Naturkautschuk auch Blockcopolymere im Verhältnis von ca. 1:1 eingesetzt werden, stellt im wesentlichen eine unbefriedigende Kompromißlösung dar, da weder hohe Scherfestigkeiten beim Einsatz der Selbstklebebänder bei höheren Temperaturen, noch deutliche Verbesserungen gegenüber der im Patent beschriebenen Eigenschaften ergibt.

Der Einsatz von ausschließlich nicht-thermoplastischen Kautschuken als Elastomerkomponente in der Haftkleberrezeptierung mit dem bestehenden Kostenvorteil, den zum Beispiel Naturkautschuke gegenüber den handelsüblichen Blockcopolymeren aufweisen, und den herausragenden Eigenschaften, insbesondere der Scherfestigkeit des Naturkautschuks und entsprechender Synthesekautschuke, wird auch in den Patenten WO 94 11 175 A1, WO 95 25 774 A1, WO 97 07 963 A1 und entsprechend US 5,539,033, US 5,550,175 ausführlich dargestellt.
Hierbei werden die in der Haftklebertechnik gebräuchlichen Zusätze wie Tackifier-Harze, Weichmacher und Füllstoffe beschrieben.
Das jeweils offenbarte Herstellungsverfahren basiert auf einem Doppelschneckenextruder, der bei der gewählten Prozeßführung über Mastikation des Kautschuks und anschließender stufenweiser Zugabe der einzelnen Zusätze mit einer entsprechenden Temperaturführung die Compoundierung zu einer homogenen Haftkleberabmischung ermöglicht.
Ausführlich wird der dem eigentlichen Herstellprozeß vorgeschaltete Mastikationsschritt des Kautschuks beschrieben. Er ist notwendig und charakteristisch für das gewählte Verfahren, da er bei der dort gewählten Technologie unumgänglich für die nachfolgende Aufnahme der weiteren Komponenten und für die Extrudierbarkeit der fertig abgemischten Masse ist. Beschrieben wird auch die Einspeisung von Luftsauerstoff, wie sie empfohlen wird, um die Kautschukmastikation zu beschleunigen.

Diese Verfahrensweise macht den nachfolgenden Schritt der Härtung durch Elektronenstrahlvemetzung (ESH) unumgänglich, ebenso wie den Einsatz von reaktiven Substanzen als ESH-Promotoren zum Erzielen einer effektiven Vemetzungsausbeute.

Beide Verfahrensschritte sind in den genannten Dokumenten beschrieben, die gewählten ESH-Promotoren neigen aber auch zu unerwünschten chemischen Vernetzungsreaktionen unter erhöhten Temperaturen, dies limitiert den Einsatz bestimmter klebrigmachender Harze.

Aufgabe der vorliegenden Erfindung ist es, ein Verfahren zur Verfügung zu stellen, mit dem sich Selbstklebemassen auf der Basis von Polyisobutylen, die zumindest einen pharmazeutischen Wirkstoff enthalten, lösemittelfrei kontinuierlich herstellen und gegebenenfalls inline beschichten lassen, ohne daß das Polyisobutylen eigenschaftsschädigend mastiziert wird.

Gelöst wird diese Aufgabe durch ein Verfahren, wie es im Hauptanspruch dargelegt ist. Anspruch 2 beschreibt das Verfahren mit geringfügigen Anpassungen. Gegenstand der Unteransprüche sind dabei vorteilhafte Weiterbildungen der Verfahren.

Demgemäß betrifft die Erfindung ein Verfahren zur kontinuierlichen lösungsmittelfreien und mastikationsfreien Herstellung von Selbstklebemassen auf Basis von Polyisobutylen mit mindestens einem pharmazeutischen Wirkstoff in einem kontinuierlich arbeitenden Aggregat mit einem Füll- und einem Compoundierteil, bestehend aus den folgenden Schritten
a) Vorlage eines Vorbatches aus granuliertem Polyisobutylen, mindestens einem Trennhilfsmittel und mindestens einem pharmazeutischen Wirkstoff in den Füllteil des Aggregats,
   gegebenenfalls Vorlage von niedermolekularem Polyisobutylen, Füllstoffen, Weichmachern, Tackifiern und/oder Harzen,
b) Übergabe der Vorlagekomponenten der Selbstklebemasse aus dem Füllteil in den Compoundierteil,
c) Zugabe der nicht in dem Füllteil aufgegebenen Komponenten der Selbstklebemasse in den Compoundierteil wie niedermolekulares Polyisobutylen, und gegebenenfalls Füllstoffe, Weichmacher, Tackifier und/oder Harze,
d) gegebenenfalls Zugabe von weiteren pharmazeutischen Wirkstoffen in den Compoundierteil des Aggregats,
e) Herstellung einer homogenen Selbstklebemasse im Compoundierteil und
f) Austragen der Selbstklebemasse.

Weiterhin umschreibt die Erfindung ein Verfahren zur kontinuierlichen lösungsmittelfreien und mastikationsfreien Herstellung von Selbstklebemassen auf Basis von Polyisobutylen mit mindestens einem pharmazeutischen Wirkstoff in einem kontinuierlich arbeitenden Aggregat mit einem Füll- und einem Compoundierteil, mit folgenden Schritten
a) Vorlage eines Vorbatches aus granuliertem Polyisobutylen, mindestens einem Trennhilfsmittel in den Füllteil des Aggregats,
   gegebenenfalls Vorlage von niedermolekularem Polyisobutylen, Füllstoffen, Weichmachern, Tackifiem und/oder Harzen,
b) Übergabe der Vorlagekomponenten der Selbstklebemasse aus dem Füllteil in den Compoundierteil,
c) Zugabe von mindestens einem pharmazeutischen Wirkstoff in den Compoundierteil und Zugabe der nicht in dem Füllteil aufgegebenen Komponenten der Selbstklebemasse in den Compoundierteil wie niedermolekulares Polyisobutylen und gegebenenfalls Füllstoffe, Weichmacher, Tackifier und/oder Harze,
d) Herstellung einer homogenen Selbstklebemasse im Compoundierteil und
e) Austragen der Selbstklebemasse.

Die Zugabe von mindestens einem pharmazeutischen Wirkstoff in den Compoundierteil und die erforderliche Zugabe der nicht in dem Füllteil aufgegebenen Komponenten der Selbstklebemasse in den Compoundierteil wie niedermolekulares Polyisobutylen, und gegebenenfalls Füllstoffe, Weichmacher, Tackifier und/oder Harze kann über die gesamte Länge des Compoundierteils erfolgen. Insbesondere sind mehrere Zudosierungsstellen möglich, so daß jede einzelne der Komponenten je nach Anforderung an die Prozeßführung gezielt über einen eigenen Zulauf zudosiert werden kann.

Als besonders vorteilhaft hat sich die Verwendung eines Doppelschneckenextruders mit mindestens einer Dosieröffnung, vorzugsweise zwischen zwei und sieben, und mindestens einer Entgasungsöffnung als kontinuierlich arbeitendes Aggregat herausgestellt.

Weiterhin sollte im Aggregat eine Temperatur von 150 °C nicht überschritten werden, bevorzugt nicht 140 °C, besonders bevorzugt nicht 130 °C, um eine thermische Schädigung insbesondere des oder der Wirkstoffe auszuschließen.

In einer vorteilhaften Ausführungsform des Verfahrens ist zwischen Aggregat und Beschichtungsvorrichtung eine Schmelzepumpe oder ein Extruder zur Förderung der Selbstklebemassen angeordnet.

Im zweiten, vorteilhafterweise im Verbund mit dem Compoundierschritt im Doppelschneckenextruder erfolgenden Verfahrensschritt wird die erfindungsgemäß hergestellte Selbstklebemasse mit einem Auftragswerk auf einen bahnförmigen Träger, auf eine Trennfolie oder auf ein Trennpapier lösemittelfrei beschichtet. Die Beschichtung kann vollflächig, aber auch partiell erfolgen.

Um einen definierten, luftblasenfreien Masseauftrag auf dem bahnförmigen Material zu erhalten, ist es vorteilhaft, daß die Selbstklebemasse vor Eintritt in die Beschichtungseinheit einer Entgasung unterworfen wird, was besonders wichtig ist im Falle der Verwendung von Schutzgasen während des Compoundierprozesses im Doppelschneckenextruder.
Gemäß dem Verfahren der vorliegenden Erfindung kann eine Entgasung unter Einfluß von Unterdruck oder gegen die Atmosphäre vorzugsweise in Schneckenmaschinen erfolgen.

Vorzugsweise hat die Selbstklebemasse am Austritt aus dem Aggregat eine Temperatur von weniger als 150 °C, vorzugsweise weniger als 130 °C, besonders bevorzugt weniger als 110 °C.

Zur Beschichtung auf bahnförmige Materialien eignen sich verschiedene Verfahren. Lösungsmittelfreie Selbstklebemassen lassen sich mittels einer dem Doppelschneckenextruder nachgeschalteten Extrusionsdüse beschichten. Zum Druckaufbau für die Düsenbeschichtung werden Einschneckenextruder und/oder Schmelzepumpen besonders bevorzugt, so daß die Beschichtung der bahnförmigen Trägermaterialien mit Masseaufträgen sehr geringer Schwankungsbreite erfolgen kann.

Eine weitere Möglichkeit zur Beschichtung von bahnförmigen Trägermaterialien mit der nach erfindungsgemäßen Verfahren hergestellten wirkstoffhaltigen Selbstklebemasse ist die Verwendung von Walzenbeschichtungsauftragswerken oder Mehrwalzen-Beschichtungskalandem, die vorzugsweise aus mindestens zwei Beschichtungswalzen bestehen, wobei die Selbstklebemasse bei Durchgang durch einen oder mehrere Walzenspalte vor Übergabe auf das bahnförmige Material auf die gewünschte Dicke geformt wird. Dieses Beschichtungsverfahren wird besonders dann bevorzugt, wenn eine Beschichtung mit Extrusionsdüsen allein nicht mehr die erforderte Genauigkeit im Masseauftrag liefert.
Je nach Art des zu beschichtenden bahnförmigen Trägermaterials kann die Beschichtung im Gleichlauf- oder Gegenlaufverfahren erfolgen.

Die Beschichtung ist auf Walzenbeschichtungsauftragswerken oder Mehrwalzen-Beschichtungskalandem bei Temperaturen unterhalb von 150 °C möglich, so daß auch Selbstklebemassen beschichtet werden können, die thermisch empfindliche Wirkstoffe enthalten. Zum Zwecke erhöhter Gasblasenfreiheit der beschichteten Klebmasse kann zwischen Doppelschneckenextruder und Auftragswerk eine Vakuumentgasung, zum Beispiel eine Vakuumkammer, ein Entgasungsextruder, Luftrakel oder ähnliches installiert sein.

Gemäß dem Verfahren vorliegender Erfindung erfolgt keine eigenschaftsschädigende Mastikation des Polyisobutylens, da kurz nach dessen Vorlage die Zugabe der Flüssigkomponenten erfolgt. Diese Flüssigkomponenten können sowohl niedermolekulares Polyisobutylen, Weichmacher und/oder Tackifier sein, aber auch Harze, die erst während des Compoundierprozesses bei Einwirken von Scherenergie und/oder äußerer Temperierung aufschmelzen. Durch die Anwesenheit dieser Flüssigkomponenten wird das Ausmaß an Friktionsenergie derartig limitiert, daß die Mastikation des Polyisobutylens sowie hohe resultierende Compoundierungstemperaturen vermieden werden können. Allerdings können in den einzelnen Verfahrensschritten Degradationsprozesse auftreten, die die Eigenschaften der Selbstklebemasse aber nicht nachhaltig verschlechtem.

Der Doppelschneckenextruder sollte über einen oder vorzugsweise mehrere separate Temperier- beziehungsweise Kühlkreise verfügen, um ein Temperaturregime zu ermöglichen, das den Einsatz thermisch empfindlicher pharmazeutischer Wirkstoffe erlaubt. In Fällen, wo dies nicht erforderlich ist, können die Temperierkreise auch miteinander verbunden werden, um die Anzahl an Temperiergeräten möglichst gering zu halten.

Das erfindungsgemäße Verfahren erlaubt die Herstellung von Selbstklebemassen mit pharmazeutischen Wirkstoffen und insbesondere im Verbund mit einer nachgeschalteten Beschichtungseinheit die Herstellung von selbstklebend ausgerüsteten Artikeln, die ihrerseits zur Herstellung von Pflastern oder Binden eingesetzt werden, unter Erlangung besonderer Kostenvorteile.

Das Verfahren besteht im wesentlichen aus den bereits dargelegten Verfahrensschritten, welche optional unter einer Schutzgasatmosphäre zur Vermeidung von oxidativem Polymerabbau durchgeführt werden können.

Im Compoundierungsschritt wird eine Masse aus Polyisobutylenen, einem oder mehreren pharmazeutischen Wirkstoffen und den benötigten Zuschlagstoffen wie niedermolekulares Polyisobutylen, Füllstoffe, Weichmacher, Tackifier und/oder Harze bevorzugt in einem Doppelschneckenextruder lösungsmittelfrei hergestellt, wobei die Masse eine Endtemperatur von weniger als 150 °C, bevorzugt weniger als 130 °C, ganz besonders bevorzugt weniger als 110 °C aufweist. Die pharmazeutischen Wirkstoffe können dabei unmittelbar zu Beginn des Verfahrens zugesetzt werden, können aber auch - je nach Empfindlichkeit des Wirkstoffes - erst zu einem späteren Zeitpunkt in den Doppelschneckenextruder gegeben werden. Die Zugabe kann in reiner oder gelöster Form erfolgen.

Erfindungsgemäß basiert die Selbstklebemasse auf Polyisobutylen.
Nach Römpp Lexikon Chemie (Version 2.0, Stuttgart/New York: Georg Thieme Verlag 1999) werden als Polyisobutene beziehungsweise Polyisobutylene (PIB) zu den Polyolefinen zählende Polymere der Struktur 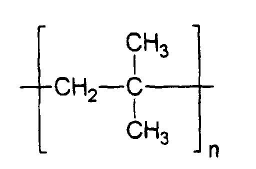 bezeichnet, die durch kationische Polymerisation von Isobuten hergestellt werden. Handelsübliche PIBs lassen sich in 3 Produktkategorien einordnen:
- ölige Flüssigkeiten (Molmasse: 300-3000 g/mol)
- zähflüssige, klebrige Massen (Molmasse: 40 000-120 000 g/mol)
- kautschukartige, elastische Massen (Molmasse: 300 000-2 500 000 g/mol)

Die unterschiedlichen Molmassen können über den Einsatz von Reglern (zum Beispiel 2,4,4-Trimethyl-1-penten, "Diisobuten") bei der Polymerisation eingestellt werden.

Zur Modifizierung der PIB-Eigenschaften kann Isobuten mit geeigneten Copolymeren [zum Beispiel Styrol und Styrol-Derivate, Isopren, Inden, 1,3-Butadien, Cyclopentadien u. a.] copolymerisiert werden. Die technisch interessanten Copolymere enthalten in der Regel >90% Isobuten.

Der Anteil des hochmolekularen, granulierten Polyisobutylens beträgt insbesondere zwischen 5 Gew.-% und 30 Gew.-%, bevorzugt zwischen 10 Gew.-% und 20 Gew.-%.
Der Anteil des niedermolekularen Polyisobutylens beträgt insbesondere zwischen 20 Gew.-% und 60 Gew.-%, bevorzugt zwischen 30 Gew.-% und 50 Gew.-%.

Als Füllstoffe kommen rieselfähige Schüttgüter sowie deren Mischungen in Frage, wie zum Beispiel Cellulose, Kieselsäure, Alginate, Pektine, die nicht in der Klebmatrix löslich sind.
Der Füllstoffanteil liegt insbesondere zwischen 0 Gew.-% und 60 Gew.-%, bevorzugt zwischen 0 Gew.-% und 40 Gew.-%, besonders bevorzugt zwischen 10 Gew.-% und 40 Gew.-%.

Als klebrigmachende Harze sind ausnahmslos alle vorbekannten und in der Literatur beschriebenen Klebharze einsetzbar. Genannt seien stellvertretend die Kolophoniumharze, deren disproportionierte, hydrierte, polymerisierte, veresterte Derivate und Salze, die aliphatischen und aromatischen Kohlenwasserstoffharze, Terpenharze und Terpenphenolharze. Beliebige Kombinationen dieser und weiterer Harze können eingesetzt werden, um die Eigenschaften der resultierenden Klebmasse wunschgemäß einzustellen. Auf die Darstellung des Wissensstandes im "Handbook of Pressure Sensitive Adhesive Technology" von Donatas Satas (van Nostrand, 1989) sei ausdrücklich hingewiesen.
Der Harzanteil liegt insbesondere zwischen 0 Gew.-% und 50 Gew.-%, bevorzugt zwischen 0 Gew.-% und 40 Gew.-%, besonders bevorzugt zwischen 10 Gew.-% und 40 Gew.-%.

Als Tackifier kommen alle bekannten klebrigmachenden Polymere in Frage, z.B. aus der Gruppe der Polyisoprene, Polybutadiene und Polyacrylate. Der Tackifieranteil liegt insbesondere zwischen 0 Gew.-% und 50 Gew.-%, bevorzugt zwischen 0 Gew.-% und 40 Gew.-%, besonders bevorzugt zwischen 5 Gew.-% und 30 Gew.-%.

Als Weichmacher können alle bekannten weichmachenden Substanzen sowie pharmazeutische Hilfsstoffe eingesetzt werden. Dazu zählen unter anderem die paraffinischen und naphthenischen Öle, (funktionalisierte) Oligomere wie Oligobutadiene, -isoprene, flüssige Nitrilkautschuke, flüssige Terpenharze, pflanzliche und tierische Öle und Fette, Fettsäureester, Phthalate, Alkohole, funktionalisierte Acrylate.
Der Weichmacheranteil liegt insbesondere zwischen 0 Gew.-% und 30 Gew.-%, bevorzugt zwischen 2 Gew.-% und 20 Gew.-%.

Als pharmazeutische Wirkstoffe werden Substanzen verstanden, die in menschlichen oder tierischen Organismen zur Verhütung, Heilung, Linderung oder Erkennung von Krankheiten dienen. Die eingesetzten pharmazeutischen Wirkstoffe können sowohl systemisch als auch lokal wirksam sein.
Typische, erfindungsgemäß eingesetzte Wirkstoffe sind hierbei:
Aceclidin, Amfetaminil, Amfetamin, Amylnitrit, Apophedrin, Atebrin, Alpostadil, Azulen, Arecolin, Anethol, Amylenhydrat, Acetylcholin, Acridin, Adenosintriphosphorsäure, L-Äpfelsäure, Alimemazin, Allithiamin, Allyl Isothiocyanat, Aminoethanol, Apyzin, Apiol, Azatadin, Alprenolol, Äthinazon, Benzoylperoxid, Benzylalkohol, Bisabolol, Bisnorephedrin, Butacetoluid, Benactyzin, Campher, Colecalciferol, Chloralhydrat, Clemastin, Chlorobutanol, Capsaicin, Cyclopentamin, Clobutinol, Chamazulen, Dimethocain, Codein, Chloropromazin, Chinin, Chlorthymol, Cyclophosphamid, Cinchocain, Chlorambuzil, Chlorphenesin, Diethylethan, Divinylethan, Dexchlorpheniramin, Dinoproston, Dixyrazin, Ephedrin, Ethosuximid, Enallylpropymal, Emylcamat, Erytroltetranitrat, Emetin, Enfluran, Eucalyptol, Etofenamat, Ethylmorphin, Fentanyl, Fluanison, Guajazulen, Halothan, Hyoscyamin, Histamin, Fencarbamid, Hydroxycain, Hexylresorcin, Isoaminilcitrat, Isosorbiddinitrat, Ibuprofen, Jod, Jodoform, Isoaminil, Lidocain, Lopirin, Levamisol, Methadon, Methyprylon, Methylphenidat, Mephenesin, Methylephedrin, Meclastin, Methopromazin, Mesuximid, Nicethamid, Norpseudoephedrin, Menthol, Methoxyfluran, Methylpentinol, Metixen, Mesoprostol, Oxytetracain, Oxyprenolol, Oxyphenbutazon, Oxychinolin, Pinen, Prolintan, Procyclidin, Piperazin, Pivazid, Phensuximid, Procain, Phenindamin, Promethazin, Pentetrazol, Profenamin, Perazin, Phenol, Pethidin, Pilocarpin, Prenylamin, Phenoxybenzamin, Resochin, Scopolamin, Salicylsäureester, Spartein, Trichlorethylen, Timolol, Trifluperazin, Tetracain, Trimipramin, Tranylcypromin, Trimethadion, Tybamat, Thymol, Thioridazin, Valproinsäure, Verapamil, sowie weitere, dem Fachmann geläufige, über die Haut, eingeschlossen Schleimhäute, aufnehmbare Wirkstoffe. Selbstverständlich ist diese Aufzählung nicht abschließend.

Der Wirkstoffanteil in der Selbstklebemasse liegt vorzugsweise zwischen 0,001 Gew.-% und 60 Gew.-% beträgt, bevorzugt zwischen 0,001 Gew.-% und 20 Gew.-%, besonders bevorzugt zwischen 0,001 Gew.-% und 10 Gew.-%.

Als bahnförmige Trägermaterialien für die erfindungsgemäß hergestellten und verarbeiteten Selbstklebemassen sind je nach Verwendungszweck alle bekannten Träger, gegebenenfalls mit entsprechender chemischer oder physikalischer Oberflächenvorbehandlung der Streichseite sowie antiadhäsiver physikalischer Behandlung oder Beschichtung der Rückseite geeignet. Genannt seien beispielsweise gekreppte und ungekreppte Papiere, Polyethylen-, Polypropylen-, mono- oder biaxial orientierte Polypropylenfolien, Polyester-, PVC- und andere Folien, bahnförmige Schaumstoffe, beispielsweise aus Polyethylen und Polyurethan, Gewebe, Gewirke und Vliese.
Schließlich kann das bahnförmige Material ein beidseitig antiadhäsiv beschichtetes Material sein wie ein Trennpapier oder eine Trennfolie. Gegebenfalls wird das beschichtete Trägermaterial mittels einer weiteren Trennfolie oder einem weiteren Trennpapier eingedeckt.

Die Dicke der Selbstklebemasse auf dem bahnförmigen Material kann zwischen 10 µm und 2000 µm betragen, vorzugsweise zwischen 100 µm und 500 µm.

Anhand der folgenden Beispiele soll die Erfindung näher beschrieben werden, ohne damit die Erfindung einschränken zu wollen.

### Beispiel 1

Zur Herstellung eines Prototypen diente ein Doppelschneckenextruder der Firma Berstorff mit 25 mm Schneckendurchmesser, wobei folgende Beispielrezeptur A angewendet wurde:

| Nr. | Komponente | | Gewichtsanteil in % |
|---|---|---|---|
| 1 | Vistanex® MM L-80 | Hochmolekulares Polyisobutylen | 12 |
| 2 | Vistanex® LM MH | Niedermolekulares Polyisobutylen | 38 |
| 3 | Hi-Sil® 233 D | Füllstoff | 14,5 |
| 4 | AVICEL® PH101 | Füllstoff | 14,5 |
| 5 | Escorez® E-1310 | Harz | 14 |
| 6 | Whitemor WOM 14 | Weichmacher | 2 |
| 7 | Ibuprofen | Pharmazeutischer Wirkstoff | 5 |

Komponente 1 wurde granuliert und mit 5 Gew.-% der Komponente 4 als Trennmittel versehen. Aus den Komponenten 1, 3, 4, 5, 6 und 7 wurde ein homogener Vorbatch erzeugt.

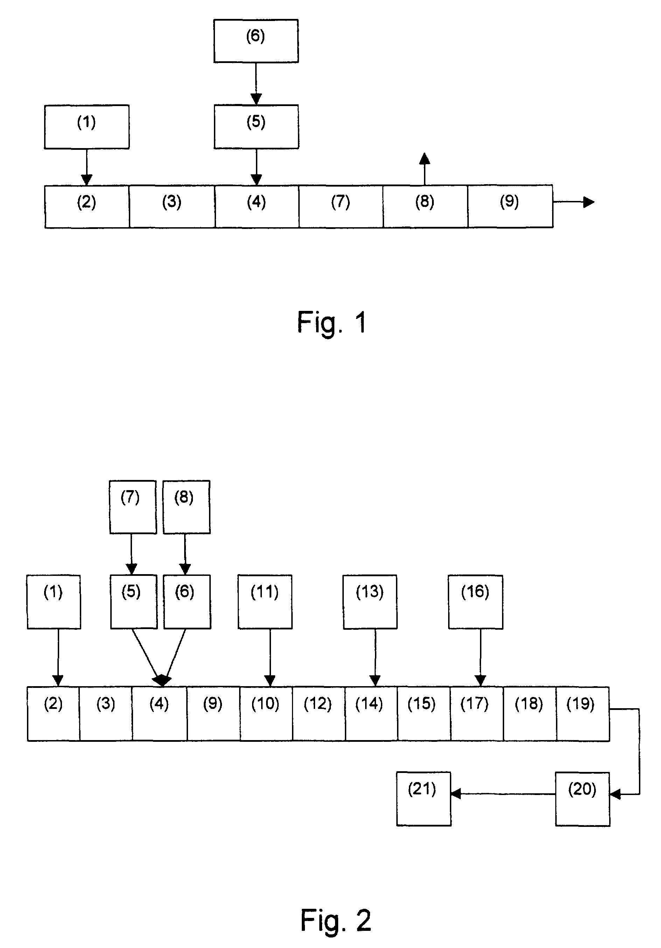

In der Figur 1 ist eine schematische Übersicht über die zur Durchführung des Verfahrens verwendete Anlage gezeigt.

Der Vorbatch wurde über eine gravimetrische Dosierung (1) dem Füllteil eines Doppelschneckenextruders zugeführt.
Das Material wurde über eine erste fördernde Verfahrenszone (2) einer zweiten (3) zugeführt, die das Material mischte.
Danach folgte eine dritte Verfahrenszone (4), die das Material förderte und der die Komponente 2 über eine volumetrisch arbeitende Zahnradpumpe (5) zudosiert wurde.
Die Zahnradpumpe wurde von einem auf 100 °C temperierten und druckbeaufschlagten (p = 6 bar) Behälter gespeist (6).
In der vierten Verfahrenszone (7) wurde das Material gemischt.
Um Luft und niedrig siedende Mischungsbestandteile zu entfernen, wird eine Entgasungszone (8) benutzt.
Die Zone (9) dient zum nochmaligen Homogenisieren und zum Druckaufbau zur Ausformung der Selbstklebemasse aus einer Düse.
Zur Vermeidung einer Schädigung des pharmazeutischen Wirkstoffs wurden alle Verfahrenszonen auf 20 °C temperiert.
Die Drehzahl des Extruder betrug 125 rpm. Am Austritt aus dem Extruder hatte die Masse eine Temperatur zwischen 75 °C und 85 °C.

### Beispiel 2

Zur Herstellung eines Prototypen diente ein Doppelschneckenextruder der Firma Leistritz mit 50 mm Schneckendurchmesser, wobei folgende Beispielrezeptur B angewendet wurde:

| Nr. | Komponente | | Gewichtsanteil in % |
|---|---|---|---|
| 1 | Vistanex® MM L-80 | Hochmolekulares Polyisobutylen | 15 |
| 2 | Vistanex® LM MH | Niedermolekulares Polyisobutylen | 29 |
| 3 | AVICEL® PH101 | Füllstoff | 35 |
| 4 | Hyvis 2000 | Tackifier | 6 |
| 5 | Cetiol® V | Weichmacher | 10 |
| 6 | Ibuprofen | Pharmazeutischer Wirkstoff | 5 |

Komponente 1 wurde granuliert und mit 5 Gew.-% der Komponente 3 als Trennmittel versehen.

In der Figur 2 ist eine schematische Übersicht über die zur Durchführung des Verfahrens benötigte Anlage gezeigt.

Das Granulat wurde über eine gravimetrische Dosierung (1) dem Füllteil des Doppelschneckenextruders zugeführt.
Das Material wurde über eine erste fördernde Verfahrenszone (2) einer zweiten (3) zugeführt, die das Material mischte.
Danach folgte eine dritte Verfahrenszone (4), die das Material förderte und der die Komponenten 2 und 4 über volumetrisch arbeitende Zahnradpumpen (5)+(6) zudosiert wurden. Die Zahnradpumpen wurden von auf 120 °C temperierten Behältern (7,8) gespeist. Im Anschluß daran wurde das Material wieder gemischt (9).
In die nächste Verfahrenszone (10), die das Material förderte, wurde die Komponente 3 gravimetrisch zudosiert (11).
Nach einer erneuten Mischungszone (12) wurde die Komponente 5 über eine Hubkolbenpumpe (13) in eine Förderzone (14) zugegeben.

Nach einer weiteren Mischungszone (15) wurde die Komponente 6 über eine gravimetrische Dosierung (16) in eine Förderzone (17) zugegeben. Anschließend wurde das Material wieder gemischt (18).

Die Zone (19) diente zum nochmaligen Homogenisieren und zum Druckaufbau. Anschließend wurde die Selbstklebemasse über eine 350mm-Breitschlitzdüse (20) ausgeformt und ausgetragen. Der Düsenspalt betrug 300 µm. Anschließend wurde die Masse in einem Glättwerk (21) kalandriert und mit zwei PET-Folien kaschiert.

Zur Vermeidung einer Schädigung der Komponente 6 wurden die Temperaturen aller Verfahrenszonen des Extruders auf Werte zwischen 60 °C und 130 °C eingestellt.

Die Drehzahl des Extruders betrug 220 rpm. Am Austritt aus dem Extruder hatte die Masse eine Temperatur von 100 °C. Die Breitschlitzdüse war auf 100 °C temperiert.

## Patentansprüche

1. Verfahren zur kontinuierlichen lösungsmittelfreien und mastikationsfreien Herstellung von Selbstklebemassen auf Basis von Polyisobutylen mit mindestes einem pharmazeutischen Wirkstoff in einem kontinuierlich arbeitenden Aggregat mit einem Füll- und einem Compoundierteil, bestehend aus
a) Vorlage eines Vorbatches aus granuliertem Polyisobutylen, mindestens einem Trennhilfsmittel und mindestes einem pharmazeutischen Wirkstoff in den Füllteil des Aggregats, gegebenenfalls Vorlage von niedermolekularem Polyisobutylen, Füllstoffen, Weichmachern, Tackifiern und/oder Harzen,
b) Übergabe der Vorlagekomponenten der Selbstklebemasse aus dem Füllteil in den Compoundierteil,
c) Zugabe der nicht in dem Füllteil aufgegebenen Komponenten der Selbstklebemasse in den Compoundierteil wie niedermolekulares Polyisobutylen und gegebenenfalls Füllstoffe, Weichmacher, Tackifier und/oder Harze,
d) gegebenenfalls Zugabe von weiteren pharmazeutischen Wirkstoffen in den Compoundierteil des Aggregats,
e) Herstellung einer homogenen Selbstklebemasse im Compoundierteil und
f) Austragen der Selbstklebemasse.

2. Verfahren zur kontinuierlichen lösungsmittelfreien und mastikationsfreien Herstellung von Selbstklebemassen auf Basis von Polyisobutylen mit mindestes einem pharmazeutischen Wirkstoff in einem kontinuierlich arbeitenden Aggregat mit einem Füll- und einem Compoundierteil, bestehend aus
a) Vorlage eines Vorbatches aus granuliertem Polyisobutylen, mindestens einem Trennhilfsmittel in den Füllteil des Aggregats, gegebenenfalls Vorlage von niedermolekularem Polyisobutylen, Füllstoffen, Weichmachern, Tackifiern und/oder Harzen,
b) Übergabe der Vorlagekomponenten der Selbstklebemasse aus dem Füllteil in den Compoundierteil,
c) Zugabe von mindestes einem pharmazeutischen Wirkstoff in den Compoundierteil und Zugabe der nicht in dem Füllteil aufgegebenen Komponenten der Selbstklebemasse in den Compoundierteil wie niedermolekulares Polyisobutylen und gegebenenfalls Füllstoffe, Weichmacher Tackifier, und/oder Harze,
d) Herstellung einer homogenen Selbstklebemasse im Compoundierteil und
e) Austragen der Selbstklebemasse.

3. Verfahren nach den Ansprüchen 1 und 2, **dadurch gekennzeichnet, daß** das Aggregat ein Doppelschneckenextruder mit mindestens einer Dosieröffnung, vorzugsweise zwischen zwei und sieben, und mindestens einer Entgasungsöffnung ist.

4. Verfahren nach zumindest einem der vorherigen Ansprüche, **dadurch gekennzeichnet, daß** im Aggregat eine Temperatur von 150 °C nicht überschritten wird, bevorzugt nicht 140 °C, besonders bevorzugt nicht 130 °C.

5. Verfahren nach zumindest einem der vorherigen Ansprüche, **dadurch gekennzeichnet, daß** zwischen Aggregat und Beschichtungsvorrichtung eine Schmelzepumpe oder ein Extruder zur Förderung der Selbstklebemasse angeordnet ist.

6. Verfahren nach zumindest einem der vorherigen Ansprüche, **dadurch gekennzeichnet, daß** Selbstklebemasse auf ein bahnförmiges Material, auf eine Trennfolie oder auf ein Trennpapier beschichtet wird und gegebenenfalls mittels einer weiteren Trennfolie oder einem weiteren Trennpapier eingedeckt wird.

7. Verfahren nach Anspruch 6, **dadurch gekennzeichnet, daß** die Beschichtung des bahnförmigen Materials mit einer Extrusionsdüse erfolgt.

8. Verfahren nach Anspruch 6, **dadurch gekennzeichnet, daß** die Beschichtung des bahnförmigen Materials mit einem Walz- oder Kalanderwerk erfolgt, wobei die Selbstklebemasse bei Durchgang durch einen oder mehrere Walzenspalte auf die gewünschte Dicke geformt wird.

9. Verfahren nach Anspruch 6, **dadurch gekennzeichnet, daß** die Beschichtung des bahnförmigen Materials mit einer Extrusionsdüse und einem Walz- oder Kalanderwerk erfolgt, wobei die Selbstklebemasse bei Durchgang durch einen oder mehrere Walzenspalte auf die gewünschte Dicke geformt wird.

10. Verfahren nach zumindest einem der vorherigen Ansprüche, **dadurch gekennzeichnet, daß** die Selbstklebemasse am Austritt aus dem Aggregat eine Temperatur von weniger als 150 °C aufweist, vorzugsweise weniger als 130 °C, besonders bevorzugt weniger als 110 °C.

11. Verfahren nach zumindest einem der vorherigen Ansprüche, **dadurch gekennzeichnet, daß** die Dicke der Selbstklebemasse auf dem bahnförmigen Material zwischen 10 µm und 2000 µm beträgt, vorzugsweise zwischen 100 µm und 500 µm.

12. Verfahren nach zumindest einem der vorherigen Ansprüche, **dadurch gekennzeichnet, daß** der pharmazeutische Wirkstoffanteil in der Selbstklebemasse zwischen 0,001 Gew.-% und 60 Gew.-% beträgt, bevorzugt zwischen 0,001 Gew.-% und 20 Gew.-%, besonders bevorzugt zwischen 0,001 Gew.-% und 10 Gew.-%.

## Claims

1. A process for the continuous solvent-free and mastication-free production of self-adhesive compositions based on polyisobutylene with at least one active pharmaceutical substance in a continuously operating apparatus having a filling section and a compounding section, comprising the following steps:
a) feeding an initial batch comprising granulated polyisobutylene, at least one release auxiliary and at least one active pharmaceutical substance into the filling section of the apparatus; if desired, feeding low molecular mass polyisobutylene, fillers, plasticizers, tackifiers and/or resins,
b) transferring the feed components of the self-adhesive composition from the filling section to the compounding section,
c) adding the components of the self-adhesive composition that have not been introduced in the filling section, such as low molecular mass polyisobutylene and, if desired, fillers, plasticizers, tackifiers and/or resins, to the compounding section,
d) if desired, adding further pharmaceutical substances to the compounding section of the apparatus,
e) preparing a homogeneous self-adhesive composition in the compounding section, and
f) discharging the self-adhesive composition.

2. A process for the continuous solvent-free and mastication-free production of self-adhesive compositions based on polyisobutylene with at least one active pharmaceutical substance in a continuously operating apparatus having a filling section and a compounding section, comprising the following steps:
a) feeding an initial batch comprising granulated polyisobutylene, at least one release auxiliary into the filling section of the apparatus;
if desired, feeding low molecular mass polyisobutylene, fillers, plasticizers, tackifiers and/or resins,
b) transferring the feed components of the self-adhesive composition from the filling section to the compounding section,
c) adding at least one pharmaceutical substance to the compound section and adding the components of the self-adhesive composition that have not been introduced in the filling section, such as low molecular mass polyisobutylene and, if desired, fillers, plasticizers, tackifiers and/or resins, to the compounding section,
d) preparing a homogeneous self-adhesive composition in the compounding section, and
e) discharging the self-adhesive composition.

3. The process as claimed in either of claims 1 and 2, wherein the apparatus is a twin screw extruder having at least one metering port, preferably between two and seven, and at least one devolatilization port.

4. The process as claimed in at least one of the preceding claims, wherein the temperature in the apparatus does not exceed 150°C, preferably not 140°C, with particular preference not 130°C.

5. The process as claimed in at least one of the preceding claims, wherein a melt pump or an extruder for conveying the self-adhesive composition is arranged between the apparatus and the coating device.

6. The process as claimed in at least one of the preceding claims, wherein self-adhesive composition is coated onto a web-form material, onto a release film or onto a release paper and if desired is lined with a further release film or a further release paper.

7. The process as claimed in claim 6, wherein coating of the web-form material takes place using an extrusion die.

8. The process as claimed in claim 6, wherein coating of the web-form material takes place using a roller or calender unit, the self-adhesive composition shaped to the desired thickness as it passes through one or more roll nips.

9. The process as claimed in claim 6, wherein coating of the web-form material takes place using an extrusion die and a roll or calender unit, the self-adhesive composition shaped to the desired thickness as it passes through one or more roll nips.

10. The process as claimed in at least one of the preceding claims, wherein at the exit from the apparatus the self-adhesive composition has a temperature of less than 150°C, preferably less than 130°C, with particular preference less than 110°C.

11. The process as claimed in at least one of the preceding claims, wherein the thickness of the self-adhesive composition on the web-form material is between 10 µm and 2000 µm, preferably between 100 µm and 500 µm.

12. The process as claimed in at least one of the preceding claims, wherein the active pharmaceutical substance fraction in the self-adhesive composition is between 0.001% by weight and 60% by weight, preferably between 0.001% by weight and 20% by weight, with particular preference between 0.001% by weight and 10% by weight.

## Revendications

1. Procédé pour la fabrication continue sans solvant et sans mastication de masses auto-adhésives à base de polyisobutylène contenant au moins une substance active pharmaceutique dans un appareil fonctionnant en continu avec une partie de remplissage et une partie de mélange, constitué par :
a) la disposition au préalable d'un lot préalable de polyisobutylène granulé, d'au moins un adjuvant de séparation et d'au moins une substance active pharmaceutique dans la partie de remplissage de l'appareil,
le cas échéant la disposition préalable de polyisobutylène de bas poids moléculaire, de charges, de plastifiants, d'agents qui rendent adhésif et/ou de résines,
b) le transfert des composants de la masse auto-adhésive disposés au préalable de la partie de remplissage dans la partie de mélange,
c) l'addition dans la partie de mélange des composants de la masse auto-adhésive non ajoutés dans la partie de remplissage, tels que du polyisobutylène de bas poids moléculaire, et le cas échéant des charges, des plastifiants, des agents qui rendent adhésif et/ou des résines,
d) le cas échéant l'addition d'autres substances actives pharmaceutiques dans la partie de mélange de l'appareil,
e) la préparation d'une masse auto-adhésive homogène dans la partie de mélange et
f) l'évacuation de la masse auto-adhésive.

2. Procédé pour la fabrication continue sans solvant et sans mastication de masses auto-adhésives à base de polyisobutylène contenant au moins une substance active pharmaceutique dans un appareil fonctionnant en continu avec une partie de remplissage et une partie de mélange, constitué par
a) la disposition au préalable d'un lot préalable constitué de polyisobutylène granulé, d'au moins un adjuvant de séparation dans la partie de remplissage de l'appareil,
le cas échéant la disposition préalable de polyisobutylène de bas poids moléculaire, de charges, de plastifiants, d'agents qui rendent adhésif et/ou de résines,
b) le transfert des composants de la masse auto-adhésive disposés au préalable de la partie de remplissage dans la partie de mélange,
c) l'addition d'au moins une substance active pharmaceutique dans la partie de mélange et le cas échéant l'addition dans la partie de mélange des composants de la masse auto-adhésive non ajoutés dans la partie de remplissage, tels que du polyisobutylène de bas poids moléculaire et le cas échéant des charges, des plastifiants, des agents qui rendent adhésif et/ou des résines,
d) la préparation d'une masse auto-adhésive homogène dans la partie de mélange et
e) l'évacuation de la masse auto-adhésive.

3. Procédé selon les revendications 1 et 2, **caractérisé en ce que** l'appareil est une extrudeuse à double vis sans fin présentant au moins une ouverture de dosage, de préférence entre deux et sept ouvertures de dosage, et au moins une ouverture de dégazage.

4. Procédé selon au moins l'une quelconque des revendications précédentes, **caractérisé en ce que** dans l'appareil, une température de 150°C, de préférence une température de 140°C, de manière particulièrement préférée une température de 130°C n'est pas dépassée.

5. Procédé selon au moins l'une quelconque des revendications précédentes, **caractérisé en ce qu'**une pompe à masse fondue ou une extrudeuse est prévue entre l'appareil et le dispositif de revêtement pour le transport de la masse auto-adhésive.

6. Procédé selon au moins l'une quelconque des revendications précédentes, **caractérisé en ce que** la masse auto-adhésive est revêtue sur un matériau en forme de bande, sur une feuille de séparation ou sur un papier de séparation et est le cas échéant recouverte au moyen d'une autre feuille de séparation ou un autre papier de séparation.

7. Procédé selon la revendication 6, **caractérisé en ce que** le revêtement du matériau en forme de bande est réalisé avec une filière d'extrusion.

8. Procédé selon la revendication 6, **caractérisé en ce que** le revêtement du matériau en forme de bande est réalisé avec un appareil à cylindres ou à calandres, la masse auto-adhésive étant façonnée à l'épaisseur souhaitée lors du passage dans une ou plusieurs fentes de laminage.

9. Procédé selon la revendication 6, **caractérisé en ce que** le revêtement du matériau en forme de bande est réalisé avec une filière d'extrusion et un appareil à cylindres ou à calandres, la masse auto-adhésive, étant façonnée à l'épaisseur souhaitée lors du passage à travers une ou plusieurs fentes de laminage.

10. Procédé selon au moins l'une quelconque des revendications précédentes, **caractérisé en ce que** la masse auto-adhésive à la sortie de l'appareil présente une température de moins de 150°C, de préférence de moins de 130°C de manière particulièrement préférée de moins de 110°C.

11. Procédé selon au moins l'une quelconque des revendications précédentes, **caractérisé en ce que** l'épaisseur de la masse auto-adhésive sur le matériau en forme de bande est située entre 10 µm et 2000 µm, de préférence entre 100 µm et 500 µm.

12. Procédé selon au moins l'une quelconque des revendications précédentes, **caractérisé en ce que** la proportion de substance active pharmaceutique dans la masse auto-adhésive est comprise entre 0,001% en poids et 60% en poids, de préférence entre 0,001% en poids et 20% en poids, de manière particulièrement préférée entre 0,001% en poids et 10% en poids.
